(19) Europäisches Patentamt European Patent Office Office européen des brevets

(11) **EP 4 643 911 A1**

(12) **EUROPEAN PATENT APPLICATION**

(43) Date of publication:
**05.11.2025 Bulletin 2025/45**

(21) Application number: 25172873.9

(22) Date of filing: **28.04.2025**

(51) International Patent Classification (IPC):
**A61M 5/158** (2006.01)    **A61L 29/06** (2006.01)
**C08L 75/04** (2006.01)

(52) Cooperative Patent Classification (CPC):
**A61M 5/158; A61L 29/06; C08G 64/18;**
**C08L 75/04; C08L 83/10;** A61M 2205/0216;
C08G 77/448; C08G 77/458

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **01.05.2024   US 202463641349 P**
**27.03.2025   US 202519092565**

(71) Applicant: **Medtronic MiniMed, Inc.**
**Northridge, CA 91325-1219 (US)**

(72) Inventors:
• **Tong, Davy T.**
  **Northridge, 91325 (US)**
• **Zhang, Guangping**
  **Northridge, 91325 (US)**
• **DANG, Kiem H.**
  **Northridge, 91325 (US)**
• **Fusselman, Hsi C.**
  **Northridge, 91325 (US)**
• **Chattaraj, Sarnath**
  **Northridge, 91325 (US)**
• **Dianaty, Ali**
  **Northridge, 91325 (US)**

(74) Representative: **Zimmermann & Partner**
**Patentanwälte mbB**
**P.O. Box 330 920**
**80069 München (DE)**

(54) **PFAS-FREE INFUSION CANNULAS**

(57) The present disclosure relates to the use of a PFAS-free cannula in delivering a fluid medication, such as insulin, to a subcutaneous site. The cannula comprises polycarbonate polyurethane polysiloxane (PC-PU-PS), wherein the cannula is configured for subcutaneous insertion into the tissue of the individual in need thereof. The disclosure includes methods for administering insulin using the cannula and infusion devices comprising the cannula.

**1. Interface :**
▪ Needle guide used to prevent inadvertent damage to the cannula from the needle during assembly

**2. Materials :**
▪ Stiff during insertion for reliable penetration
▪ Soft in the body after insertion to minimize inflammation and injury
▪ Low friction for insertion reliability and minimal tissue trauma
▪ Coating (silicone oil) reduces effects of micromotion and increases longevity

**3. Biological Behavior:**
▪ Material unreactive to tissue
▪ Surface microstructure minimizes inflammatory response

**4. Process (Flaring):**
▪ Material compliant enough to flare
▪ Material elastic enough to form a fluid-tight seal

**5. Geometry (Length) :**
▪ Sufficiently long to reliably penetrate Sub-Q.
▪ Sufficiently short to avoid reaching muscle tissue.

**Quality of the tip is important for penetration and longevity of the catheter**

**6. Process & Geometry (Tipping):**
▪ Tip geometry optimized for minimal penetration force and pain
▪ Material compliant enough to tip into optimal geometry
▪ Tip must not be too sharp or too blunt for in-vivo longevity

**7. Fluid Path:**
▪ Material unreactive to insulin
▪ Opening sufficiently large to deliver without occlusions
▪ Opening sufficiently small to minimize tissue trauma
▪ Fill volume minimized to avoid wasted insulin

FIG. 1

**Description**

CROSS-REFERENCE TO RELATED APPLICATIONS

This application claims the benefit of priority to U.S. Provisional Application No. 63/641,349, filed May 1,2024 and 19/092,565 filed March 27, 2025 which is incorporated herein by reference in its entirety.

TECHNICAL FIELD

The present technology relates generally to medical devices, and more particularly, to PFAS-free infusion cannulas for administering medications to individuals, such as insulin to diabetic patients.

BACKGROUND

Millions of individuals having diabetes require insulin therapy to manage uncontrolled blood sugar levels (*i.e.*, blood glucose levels). As an alternative to multiple daily injections (syringe or pen injections), many people rely on small, wearable insulin infusion devices to manage their blood sugar. Typically, an infusion device includes a pump (which includes controls, a processing module, and batteries), a reservoir containing fluid medication (e.g. insulin), and an infusion set/sub-system. The infusion set/subsystem includes a cannula configured for subcutaneous insertion into the individual and a tubing system connecting the reservoir to the cannula. When the tubing in the infusion sub-system is minimal and the pump is adhered to skin, the pump system is wearable and called a patch pump. The cannula and tubing are part of the infusion sub-system in a patch pump. An infusion device where the pump is not worn against the skin but is tethered by a longer tubing is called a tethered pump, and the cannula and tubing are referred to as an infusion set. In either the patch pump or the tethered pump, the cannula is inserted subcutaneously and maintained at the infusion site for multiple days to enable delivery of the fluid medication. Cannulas provide a passageway for delivering the medication to the individual subcutaneously.

The most popular and widely used cannulas are made of polytetrafluoroethylene (PTFE) and fluoroethylenepropylene (FEP). These biocompatible polymers are lubricious and allow for ease of manufacturing. Cannulas made from these polymers can reliably deliver insulin to an individual for multiple days before performance degrades and the cannula must be removed and replaced.

However, one drawback of using PTFE and FEP polymers is that they are composed of per- and polyfluroalkyl substances (PFAS), long lasting chemicals which have been associated with adverse effects to human health and are difficult to break down. Since PFAS do not degrade easily, they can potentially build up over time and accumulate in the body and environment. Studies have revealed a correlation between exposure to certain PFAS and adverse effects on reproductive ability, developmental delays in children, an increased risk of cancers, reduction of the body's immune system to fight infections, interference with the body's natural hormones, and an increase in cholesterol levels and the risk of obesity.

There is a demand for biocompatible cannulas comprised of PFAS-free materials that are suitable for conventional manufacturing processes and can deliver insulin to the tissue of an individual over several days while maintaining good performance. A PFAS-free cannula would not only benefit millions of diabetic patients who need subcutaneous infusions of insulin, but any individual that needs subcutaneous infusions of a fluid medication.

SUMMARY

This disclosure provides a method for subcutaneous administration of insulin to diabetic patients, via a PFAS-free cannula configured for subcutaneous insertion. In addition, the cannula and infusion devices are disclosed herein.

In one aspect, the disclosure provides for a cannula for use in delivering insulin to an individual in need thereof, wherein: the cannula comprises a copolymer, and the copolymer is polycarbonate polyurethane polysiloxane (PC-PU-PS); and the cannula is configured for subcutaneous insertion into the tissue of the individual in need thereof.

In another aspect, the disclosure provides for a method of administering insulin to an individual in need thereof, comprising: providing a cannula, wherein the cannula comprises a copolymer, and the copolymer is polycarbonate polyurethane polysiloxane (PC-PU-PS), wherein the cannula is configured for subcutaneous insertion into the tissue of the individual in need thereof; inserting the cannula into the individual; and delivering insulin via the cannula into the individual in need thereof.

In yet another aspect, the disclosure provides an infusion device comprising: a housing configured to be positioned at the skin of a patient at an infusion site; a reservoir configured to store a fluid medication, the reservoir configured to be received by the housing; and a cannula, as described herein, configured for subcutaneous insertion into a tissue of the patient at the infusion site.

Various embodiments disclosed herein are provided as examples and do not limit the subject technology.

BRIEF DESCRIPTION OF THE DRAWINGS

FIG. 1 summarizes certain general aspects of an example cannula shown as flared onto a bushing or needle guide.

FIG. 2 illustrates an example of the overall cannula and optional cannula tip manufacturing process.

FIG. 3 is a photo of an example cannula with a needle inside the tipped cannula.

FIG. 4 is a plot of the elastic stiffness of three different materials as a function of temperature as measured by Dynamic Mechanical Analysis (DMA) at a cycle rate of 0.8 Hz.

FIGS. 5 are plots of the storage and loss moduli, and tan delta of polycarbonate polyurethane polysiloxane (PC-PU-PS) (FIG. 5A) and PTFE (FIG. 5B) as measured by dynamic mechanical analysis (DMA) at a cycle rate of 0.8 Hz.

FIG. 6. is a graphical summary of the TDD of insulin delivered to diabetic swine over nine days by each cannula material test group (EIS, ARCS-95A, ARCS-70D, ARCS-55D).

FIG. 7 is a drawing of a cannula example, where dimension ranges are provided in the Example section.

DETAILED DESCRIPTION

I. Cannula Overview

The cannula is a crucial component of infusion therapy and serves as the interface between the infusion device and the patient's subcutaneous tissue. A cannula provides the ability to convey fluid medication, such as insulin, to a targeted location on an individual's body (*i.e.* the infusion site) over a period of days (*e.g.* 1-2 days, 2-3 days, up to 6-7 days). Some cannulas are for subcutaneous insertion and can be 22-30 gauge. Some cannulas are made of a synthetic polymer and may also be known as a plastic catheter.

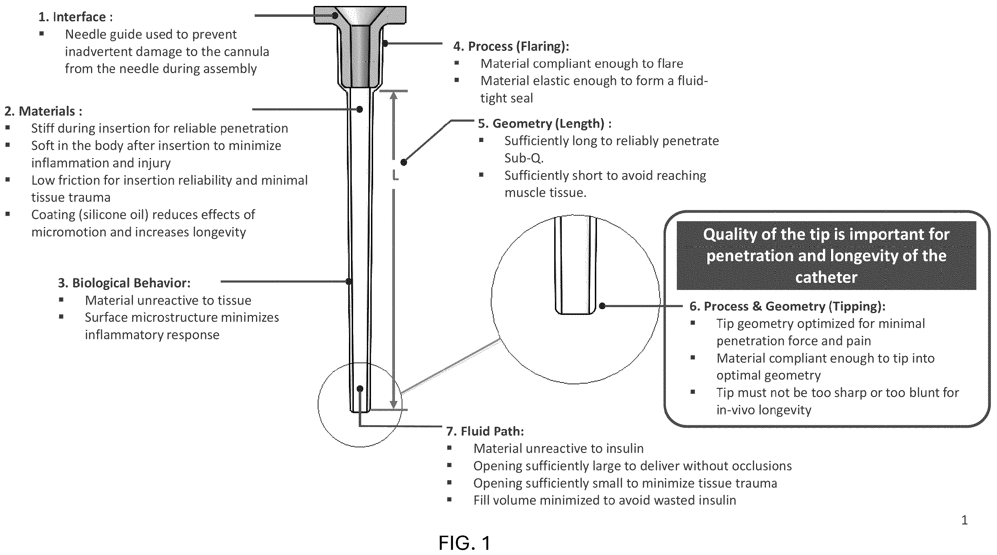

FIG. 1 is an illustration of a cannula mated to a bushing, which can function as a needle guide. The bushing connects the cannula to the rest of the infusion device. The bushing and cannula together function to provide a sealed fluid delivery path for a drug (e.g. insulin) into subcutaneous tissue of a patient (e.g. diabetic patients).

A. Foreign Body Response and Cannula-Related Complications

The implantation of a cannula in human tissue triggers an immunological response known as the foreign-body response. This response manifests as an acute inflammatory reaction localized at the insertion site and may encompass the epidermis, dermis, and subcutaneous adipose tissue. Beyond the immunological response, the insertion process itself can induce mechanical trauma. This trauma can affect cells and connective tissue along the cannula's path, potentially damaging basement membranes, the extracellular matrix, and structural proteins. Disruption of the vascular network, including lymphatic vessels, arterioles, capillaries, and venuoles, can further compromise the tissue microenvironment and lead to fluid accumulation and potential clotting.

Another complication associated with cannula implantation is infusion site-loss or site-reduction. This phenomenon is believed to be partially mediated by encapsulation of the cannula by fibrous tissue and the quality of infused insulin, which may be affected by temperature and the interaction of insulin and materials it contacts (e.g. reservoir and fluid path during storage, filling, and delivery.) The encapsulation process can lead to inconsistent and unreliable medication delivery. In the case of inconsistent insulin, an individual is likely to experience blood glucose variability. The exact mechanisms underlying site-loss/reduction remain unclear, but likely involve a complex interplay between factors like localized inflammation, intraluminal coagulation within the cannula, and progressive fibrotic tissue proliferation. In addition, the movement of the cannula during daily activities can exacerbate the host response and contribute to ongoing tissue irritation.

The immunological response, tissue trauma, and infusion site-loss described above results in the failure of the cannula, the loss of effective insulin delivery, and ultimately uncontrolled blood glucose levels. In order to effectively deliver insulin and control blood glucose levels, the infusion set is replaced which involves removing failed cannula and inserting a fresh cannula, often on a different infusion site. One way to prolong the longevity of a cannula is to reduce or minimize the foreign body response, mechanical trauma, and infusion site-loss by optimizing the cannula material and performance.

B. Elastic Stiffness

The material of the cannula is stiff to facilitate penetration and insertion into patient tissue, and is resistant to bending or kinking, which could block the flow of insulin. The cannula material is compliant enough to flare and conform to a bushing or needle guide, yet elastic enough to form a fluid-tight seal around the bushing or needle guide. The cannula material is unreactive to patient tissue.

Cannulas that vary in their stiffness as a function of temperature may reduce mechanical trauma due to micromotion in an individual over time. Such a cannula is sufficiently rigid at room temperature to penetrate and insert into patient tissue, but then as it is in tissue contact with the patient and rises to body temperature, the cannula softens and becomes more flexible after being implanted in the body. This flexibility enables the cannula to bend or otherwise accommodate any movement or micromotion and/or deform due to the tissue pressure of the individual. Compared to a stiffer material, the flexibility of the implanted cannula reduces tissue injury or trauma, thus reducing inflammation, occlusion, and infusion site-loss, which extends the useful life of the implanted cannula. Reducing these negative effects in a patient increases the duration of effective delivery of fluid medication (e.g. insulin) and longer periods of time before the infusion set (e.g. cannula) needs to be replaced with a fresh set. Reducing the number of cannula insertions that a patient must perform enhances a patient's experience with infusion therapy, which in some cases can be a life-long treatment.

The stiffness or flexibility of the cannula can be assessed by measuring the elastic stiffness of the cannula material. One way to measure elastic stiffness is by dynamic mechanical analysis (DMA), which methods are known to a person having ordinary skill in the art. Analysis by DMA can provide parameters such as storage modulus, loss modulus, tan delta, and elastic modulus, which give an indication of the material's physical character. The DMA test method utilizes the following equation to calculate the relevant characteristic:

$$E = \frac{\sigma_o}{\varepsilon_o} = \frac{E'}{cos\delta} = \frac{E'}{cos\left(tan^{-1}\left(\frac{E''}{E'}\right)\right)}$$

In the equation above, E' is the storage modulus, E" is the loss modulus, and delta is the phase shift. The raw data output from the DMA (E', E", and delta) are used compute E, the elastic modulus.

The elastic stiffness is a function of the material (e.g. PC-PU-PS) used to prepare the cannula. In some embodiments, the elastic stiffness is a measure of the elastic modulus of the material. In a stress-strain curve, the elastic modulus is calculated from the slope, which is stress/strain, of the linear region (elastic deformation) prior to permanent deformation of the material. The change in the physical characteristics of the cannula material as a function of temperature can be assessed by subjecting the material to DMA over a range of temperatures. For instance, storage and loss moduli, and tan delta data of a polycarbonate polyurethane polysiloxane (PC-PU-PS) and a PTFE cannula can be measured over a range of temperatures as discussed in the Example section. The performance of cannulas prepared from different materials can be compared by plotting their elastic stiffness (the elastic modulus) over a range of temperatures. The Example section also includes a comparison of the elastic stiffness of FEP, PTFE, and PC-PU-PS from 25 °C to almost 60 °C.

Other methods of obtaining measurements related to elasticity include performing a uniaxial tensile test on a Instron at each temperature of interest, which is a method known to a person having ordinary skill in the art.

The *in vivo* performance of a cannula is also impacted by its surface microstructure. A smooth surface reduces protein adherence to the cannula, potential occlusions, and minimizes the inflammatory response. The smooth surface also reduces friction during insertion, which helps reduce insertion force and tissue trauma. A smoother cannula surface can prolong the consistent delivery of insulin and avoid infusion site-loss for a longer period of time compared to a rougher surface. The smoothness of a surface may be evaluated by visually examining the surface with the aid of an instrument such as a microscope or SEM.

The quality of the cannula tip is critical for penetration and longevity of the cannula *in vivo.* The tip geometry of the cannula is optimized for minimal penetration force and causing minimal pain to the patient. To increase longevity of the cannula *in vivo,* the tip needs to strike a balance between being sharp and blunt. A sharp tip reduces the force needed to pierce the tissue, minimizing discomfort during insertion. After the cannula is inserted into the tissue, a degree of bluntness in the tip would help reduce tissue damage during any subsequent movement of the cannula tip within the body. Reducing the effects of micromotion of the cannula in the body by including an optional coating of silicone oil may increase cannula longevity.

The opening of the cannula is sufficiently large to deliver a fluid medication (*e.g.* insulin) without forming occlusions, and sufficiently narrow to minimize trauma to patient tissue during insertion and placement at the infusion site. In addition, the fill volume of the cannula is minimized to reduce the amount of insulin that is wasted after disposing the used cannula.

## C. General Manufacturing Process

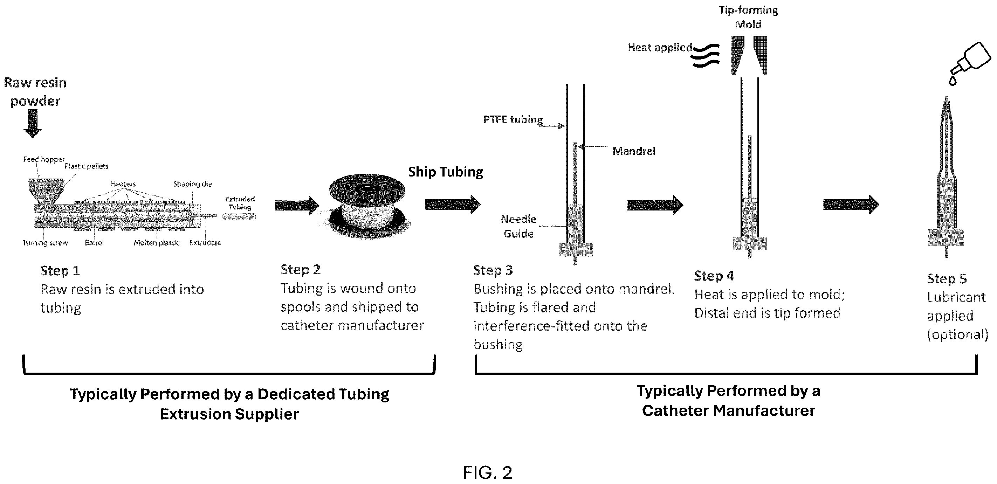

An overview of conventional cannula manufacturing and optional tip-forming process is shown in FIG. 2. The conventional cannula fabrication process begins with adding the raw materials (*i.e.* copolymer) into a feed hopper of a screw extruder machine. The screw is rotated at a predetermined speed and temperature controllers connected to heating/cooling elements on the barrel to maintain the temperature at the set-point temperatures. The extrudate leaves the die, it can either be set to the desired shape or its shape can be altered and then set to shape, and the resulting catheter is cooled. The term "catheter" may be interchangeable with "cannula" as used herein. The catheter can be extruded on conventional manufacturing equipment and specific processing parameters related to the particular resin(s) may be obtained from the supplier. The extruded catheter is then cut to length and flared onto a mandrel mounted bushing by interference fitting, which is also known as friction fit or pressed fit. The distal end of the catheter is then tip-formed using a heated and lubricated mold. The tip-forming process requires a heated mold that is typically lubricated to prevent the tubing from sticking. Finally, the tipped cannula may be treated with a lubricant.

The tip-forming mold determines the geometry of the distal portion of the cannula, features such as a rounded tip or tapered shape. The mold is designed to provide an optimized tipped catheter, where the shape and dimensions facilitate insertion into the body and reduce tissue trauma. The tip-forming process requires pre-heating a tip-forming mold to a predetermined temperature in order to reflow the resin of the tubing and force the tip of the tubing to conform to the shape of the mold. A silicone/siloxane lubricant is applied to the mold surfaces just prior to tip-forming. The distal end of the cannula (also known as a catheter tubing) is forced and pressed into the mold. A mandrel is used to hold, guide, and center the cannula with the mold during tip-forming. The distal end is held in the mold for a predetermined dwell time to allow the distal end of the cannula in contact with the mold to reflow and conform to the dimensions of the mold. After the predetermined dwell time is completed, the cannula is retracted from the mold. A successfully formed tip will have a smooth surface while also conforming to the dimensions of the molding surfaces.

In some embodiments, the cannula is a tipped cannula. In some embodiments, the cannula is not tipped.

## D. PFAS-Free Cannula Alternatives

Polytetrafluoroethylene (PTFE) and fluorinated ethylene propylene (FEP) have been the top materials of choice for cannula formation. PTFE can be reflowed when heated and is more lubricious than other resins, thus conforming to the shape of the mold and reducing the chance of sticking to the molding surfaces. Despite the lubricity of PTFE, mold surfaces are typically lubricated during fabrication in order to facilitate tip-forming and reduce sticking of the tubing to the mold.

PTFE and FEP are among the hundreds of chemicals categorized as perfluoroalkyl and polyfluoroalkyl substances (PFAS). PFAS are characterized by a chain of carbon atoms bonded to fluorine atoms and have been referred to as "forever chemicals" because of their persistence and resistance to degradation. There is a growing awareness of PFAS accumulating in the soils, water, and living organisms. The extent of the adverse impact of forever chemicals on human health and the environment is the subject of ongoing study. In the meantime, this disclosure provides for an alternative to the PTFE and FEP cannulas that are currently marketed. In some embodiments, the cannula described herein is PFAS-free.

A suitable alternative to PFAS-free materials is not obvious and there are many manufacturing and mechanical reasons why a material may not be appropriate for use in an infusion cannula. Hence the prevalence of PTFE and FEP cannula and catheters. Unlike PTFE and FEP, many other resins either do not reflow well or become extremely "sticky" when heated and adhere to molding surfaces. Resins that lack of sufficient reflow result in a cannula tip that does not properly adopt the form of the mold and has poor quality features. The term "reflow" refers to the process where the cannula material melts during the tip-forming process and conforms to the dimensions of the mold, then solidifying when cooled. Some materials are inferior because they do not possess sufficient stiffness and will result in the cannula kinking or buckling during insertion, thus damaging the cannula. Some materials may not be sufficiently soft, which can lead to tissue trauma and inflammation due to the cannula moving while implanted in the body. Other resins might not be biocompatible and may react with the patient's tissue.

## E. Copolymer

In some embodiments, the disclosure provides for a cannula comprising a PFAS-free copolymer, wherein the cannula is configured for subcutaneous insertion into the tissue of the individual in need thereof. The copolymer is compatible with conventional manufacturing processes, possesses sufficient stiffness to withstand insertion forces without bending or kinking, is sufficiently soft to reduce tissue trauma and inflammation, and is biocompatible.

The term "copolymer" refers to any polymer formed from two or more monomers. An example of a copolymer includes polycarbonate polyurethane polysiloxane (PC-PU-PS). The polycarbonate component of the copolymer is known to be a rigid, hard, and stiff material. Polycarbonates are commonly chosen in the construction and automotive industry for their durability and are utilized in items such as roofing sheets, headlight covers, and windows. The incorporation of this component in the PC-PU-PS copolymer for use as a cannula material is a surprising and unusual choice. A person having ordinary skill in the art would be unlikely to choose polycarbonate as a component of a cannula material because its inflexibility and hardness has the potential to cause discomfort in the patient and injury to the tissue, leading to adverse events such as inflammation, foreign body response, and infusion site-loss.

In one embodiment, the raw material used in the preparation of the cannula is a PC-PU-PS resin. PC-PU-PS resin is available from the manufacturer Biomerics under the tradename Quadrasil™ ARCS. Quadrasil™ ARCS is a family of aromatic polycarbonate polyurethane polysiloxane copolymers. These copolymers may also be described as aromatic polycarbonate silicone TPU. A list of nine Quadrasil™ ARCS copolymers are listed in Table 1 along with their physical properties.

Table 1.

| Property | ASTM Test | ARCS-70A | ARCS-75A | ARCS-80A | ARCS-85A | ARCS-90A | ARCS-95A | ARCS-55D | ARCS-60D | ARCS-70D |
|---|---|---|---|---|---|---|---|---|---|---|
| Durometer Hardness | D2240 | 70A | 75A | 80A | 85A | 90A | 95A | 55D | 60D | 70D |
| Specific Gravity | D792 | 1.15 | 1.15 | 1.16 | 1.16 | 1.17 | 1.17 | 1.18 | 1.20 | 1.20 |
| Flex Modulus (psi) | D790 | 700 | 1000 | 1500 | 3000 | 5500 | 10000 | 25000 | 38000 | 55000 |
| Ultimate tensile (psi) | D412 | 3500 | 4000 | 4800 | 5500 | 5800 | 6000 | 6500 | 6800 | 7300 |
| Ultimate Elongation (%) | D412 | 500 | 475 | 450 | 425 | 400 | 380 | 350 | 300 | 200 |
| Tensile at 100% (psi) | D412 | 550 | 700 | 1200 | 2100 | 2200 | 2400 | 2800 | 3100 | 3900 |
| Tensile at 300% (psi) | D412 | 1900 | 2200 | 2800 | 3100 | 3500 | 4500 | 4800 | 6800 | N/A |
| Mold Shrinkage (in/in) | D955 | .008-.012 | .008-.012 | .008-.012 | .008-.012 | .008-.012 | .008-.012 | .008-.012 | .008-.012 | .008-.012 |

A PC-PU-PS copolymer such as Quadrasil™ ARCS, has measurable physical properties such as hardness, specific gravity, flex modulus, ultimate tensile, ultimate elongation, tensile at 100%, tensile at 300%, and mold shrinkage, as listed in Table 1. These properties may be measured by standardized tests, for example, those developed by ASTM International.

In some embodiments, the polycarbonate polyurethane polysiloxane copolymer is Quadrasil™ ARCS, which may be obtained from a commercial supplier such as Biomerics as a resin. In some embodiments, the polycarbonate polyurethane polysiloxane copolymer is selected from the group consisting of ARCS-70A, ARCS-75A, ARCS-80A, ARCS-85A, ARCS-90A, ARCS-95A, ARCS-55D, ARCS-60D, and ARCS-70D, or a combination thereof. In some embodiments, the polycarbonate polyurethane polysiloxane copolymer is selected from the group consisting of ARCS-90A, ARCS-95A, ARCS-55D, ARCS-60D, and ARCS-70D, or a combination thereof. In some embodiments, the polycarbonate polyurethane polysiloxane copolymer is selected from the group consisting of ARCS-95A and ARCS-70D, or a combination thereof.

In some embodiments, the polycarbonate polyurethane polysiloxane copolymer disclosed herein has a physical property profile comprising two or more physical properties listed in Table 1, e.g. hardness, specific gravity, flex modulus, ultimate tensile, ultimate elongation, tensile at 100%, tensile at 300%, and mold shrinkage, wherein the physical property profile is equivalent to the physical property profile of a Quadrasil™ ARCS polycarbonate polyurethane polysiloxane copolymer, described in Table 1.

In some embodiments, the copolymer disclosed herein has a physical property profile comprising two or more physical properties selected from the group consisting of durometer hardness, specific gravity, flex modulus, ultimate tensile, ultimate elongation tensile at 100%, tensile at 300%, and mold shrinkage, wherein the physical property profile is equivalent to the physical property profile of an ARCS copolymer described in Table 1.

In some embodiments, the copolymer disclosed herein has a physical property profile comprising two or more physical properties selected from the group consisting of durometer hardness, specific gravity, flex modulus, ultimate tensile, ultimate elongation tensile at 100%, tensile at 300%, and mold shrinkage, wherein the physical property profile is equivalent to the physical property profile of an ARCS copolymer selected from the group consisting of ARCS-90A, ARCS-95A, ARCS-55D, ARCS-60D, and ARCS-70D. In some embodiments, the physical property profile is composed of three or more, four or more, five or more, six or more, or seven or more of the aforementioned physical properties.

In some embodiments, the copolymer disclosed herein may have a physical property profile equivalent to the physical property profile of ARCS-95A or ARCS-70D. For instance, in some embodiments, the copolymer has two or more physical properties selected from the group consisting of: durometer hardness of 95A, specific gravity of about 1.17, flex modulus of about 10000 psi, ultimate tensile of about 6000 psi, ultimate elongation of about 380%, tensile at 100% of about 2400 psi, tensile at 300% of about 4500 psi, and mold shrinkage of about .008 - .012 in/in. In some embodiments, the copolymer has two or more physical properties selected from the group consisting of: durometer hardness of about 70D, specific gravity of about 1.20, flex modulus of about 55000 psi, ultimate tensile of about 7300 psi, ultimate elongation of about 200%, tensile at 100% of about 3900 psi, and mold shrinkage of about .008 - .012 in/in. In some embodiments, the copolymer has three or more, four or more, five or more, six or more, or seven or more of the aforementioned physical properties.

The term "equivalent", as used herein, is plus or minus 0.1% to 20%, plus or minus 0.1% to 10%, plus or minus 0.1% to 5%, or plus or minus 0.1% to 2% of the values listed in Table 1.

F. Cannulas Configured with Needles

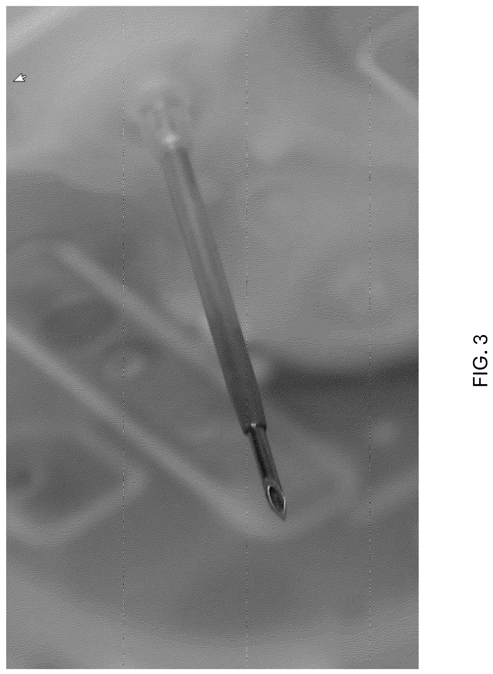

A needle may optionally be used to facilitate insertion of a cannula by puncturing the tissue (e.g. skin) of an individual in order to allow a cannula to be inserted and positioned at the intended site. A cannula can be configured to surround the outer surfaces of a needle. For instance, FIG. 3 shows a photo of a needle inside the lumen of a tipped cannula. Alternatively, the needle can be configured to surround the outer surfaces of the cannula.

In some embodiments, the cannula further comprises a needle located within the cannula. In some embodiments, the cannula further comprises a needle located on the exterior of the cannula. In embodiments, where the cannula further comprises a needle located on the exterior of the cannula, the cannula is not tipped. In some embodiments, the cannula further comprises a needle located within the cannula and the cannula is a tipped cannula. In some embodiments, the cannula is configured for subcutaneous insertion into a tissue of a diabetic patient. In some embodiments, the cannula is part of an infusion set/subsystem.

In some embodiments, the cannula is for use in delivering insulin to an individual in need thereof, wherein: the cannula comprises a copolymer, and the copolymer is polycarbonate polyurethane polysiloxane (PC-PU-PS); the cannula is configured for subcutaneous insertion into the tissue of the individual in need thereof; the difference in elastic stiffness of the cannula at 25 °C and 37 °C is at least 150 MPa as measured by DMA at a cycle rate of 0.8 Hz; and the copolymer has a physical property profile equivalent to the physical property profile of an ARCS copolymer selected from the group consisting of ARCS-90A, ARCS-95A, ARCS-55D, ARCS-60D, and ARCS-70D; and the ARCS copolymer physical property profile is listed in Table 1.

In some embodiments, the cannula is for use in delivering insulin to an individual in need thereof, wherein: the cannula comprises polycarbonate polyurethane polysiloxane copolymer (PC-PU-PS); the cannula is configured for subcutaneous insertion into the tissue of the individual in need thereof; and the difference in elastic stiffness of the cannula at 25 °C and 37 °C is at least 150 MPa as measured by DMA at a cycle rate of 0.8 Hz.

In some embodiments, the cannula is for use in delivering insulin to an individual in need thereof, wherein: the cannula comprises polycarbonate polyurethane polysiloxane copolymer (PC-PU-PS); the cannula is configured for subcutaneous insertion into the tissue of the individual in need thereof; and the elastic stiffness of the cannula at 25 °C is at least 50% greater than the elastic stiffness of the copolymer at 37 °C, as measured by DMA at a cycle rate of 0.8 Hz.

In some embodiments, the cannula is for use in delivering insulin to an individual in need thereof, wherein: the cannula comprises polycarbonate polyurethane polysiloxane copolymer (PC-PU-PS); the cannula is configured for subcutaneous insertion into the tissue of the individual in need thereof; the difference in elastic stiffness of the cannula at 25 °C and 37 °C is at least 150 MPa, as measured by DMA at a cycle rate of 0.8 Hz; and the copolymer has a physical property profile comprising two or more physical properties selected from the group consisting of durometer hardness, specific gravity, flex modulus, ultimate tensile, ultimate elongation tensile at 100%, tensile at 300%, and mold shrinkage; and the physical property profile is equivalent to the physical property profile of a copolymer selected from the group consisting of ARCS-90A, ARCS-95A, ARCS-55D, ARCS-60D, and ARCS-70D, as described in Table 1.

In some embodiments, the cannula is for use in delivering insulin to an individual in need thereof, wherein: the cannula comprises polycarbonate polyurethane polysiloxane copolymer (PC-PU-PS); the cannula is configured for subcutaneous insertion into the tissue of the individual in need thereof; the elastic stiffness of the cannula at 25°C is at least 50% greater than the elastic stiffness of the copolymer at 37 °C, and the copolymer has a physical property profile comprising two or more physical properties selected from the group consisting of durometer hardness, specific gravity, flex modulus, ultimate tensile, ultimate elongation tensile at 100%, tensile at 300%, and mold shrinkage; and the physical property profile is equivalent to the physical property profile of a copolymer selected from the group consisting of ARCS-90A, ARCS-95A, ARCS-55D, ARCS-60D, and ARCS-70D, as described in Table 1.

## II. Methods of Using a Cannula to Deliver Fluid Medications

The disclosure provides for a method of using a cannula described herein to administer a fluid medication to an individual in need thereof. The individual in need thereof refers to a subject in need of a subcutaneous infusion of a fluid medication (e.g. insulin), and includes humans, pigs, dogs.

The fluid medication can be any medication where subcutaneous delivery is desired. Examples of subcutaneous medications include insulin, growth hormone, epinephrine, opioids, heparin, and allergy medications. Medications that require prolonged delivery to an individual over a period of days can benefit from use of the cannula.

Administration of a fluid medication may be carried out with an infusion device that contains the cannula. The infusion device includes a pump (which includes controls, a processing module, and batteries), a reservoir containing fluid medication (e.g. insulin), and an infusion set/subsystem, which includes the cannula described herein for subcutaneous insertion into the individual and a tubing system connecting the reservoir to the cannula. An infusion set refers to both an external infusion set connecting a tethered pump through a long tubing to a cannula, as well as an internal infusion set such as within a body worn patch pump (*i.e.*, the pump, cannula and tubing are all within the patch device).

In some embodiments, the disclosure provides a method of administering insulin to an individual in need thereof, comprising providing a cannula as described herein; inserting the cannula into the individual; and delivering insulin via the cannula into the individual in need thereof.

The cannula is inserted subcutaneously at an infusion site located on the abdomen, thighs, hips, upper arms, lower back, or buttocks of the individual in need thereof. During insertion into the individual in need thereof, the cannula resists bunching or kinking. Insertion results in the cannula being implanted in the individual. The individual can continue to "wear" the cannula during this period of implantation. The term "to wear" as used herein, refers to having the cannula implanted in the individual after insertion.

Once implanted, the cannula is in contact with (*i.e.* implanted in) the individual in need thereof for at least 3, at least 4, at least 5, at least 6, at least 7, at least 8, at least 9, or at least 10 continuous days before the cannula is replaced or removed. The implantation of the cannula is maintained as long as the flow of the fluid medication (*e.g.* insulin) is sufficient to benefit the individual being administered the medication. In an individual with diabetes, the beneficial administration of insulin results in the effective control of blood glucose levels. When foreign body responses, inflammatory reactions, clotting, or any other adverse reaction occurs to cause the infusion site to fail, the blood glucose level will increase despite administration of larger doses of insulin. This is a signal that the individual no longer benefits from the infusion therapy and the cannula should be removed with a fresh cannula inserted at a different infusion site. Currently, manufacturers recommend removing the cannula and inserting it at a different site on the body every 3 days, and some cannulas up to 7 days. Exceeding this time increases the chance for tissue damage, infection, scarring, and increase in blood glucose.

In some embodiments the individual in need thereof is a human. In some embodiments, the individual is a diabetic human. In some embodiments, the individual is a diabetic swine.

In some embodiments, the distal end of the cannula comprises a tip configured to penetrate the skin of the individual in need thereof. In some embodiments, the cannula is configured to be worn (*i.e.* implanted) for at least 2 days, at least 3 days, or at least 3 days at a single site on a diabetic patient. In some embodiments, the cannula is in contact with the individual in need thereof for at least 3, at least 4, at least 5, at least 6, at least 7, at least 8, at least 9, or at least 10 continuous days before the cannula is replaced or removed.

In some embodiments, the cannula is used to deliver a fluid medication to an individual in need thereof. In some embodiments, the cannula is used to deliver insulin. In some embodiments, the cannula is a fluid conduit for a fluid medication. In some embodiments, the cannula is used to deliver insulin to a diabetic subject at a single site of infusion over a period of time (*e.g.* 1-2 days, 2-3 days, at least 6-7 days, at least 10 days).

In some embodiments, the disclosure provides a method of administering insulin to an individual in need thereof, comprising: providing a cannula, wherein the cannula comprises a copolymer, and the copolymer is polycarbonate polyurethane polysiloxane (PC-PU-PS), wherein the cannula is configured for subcutaneous insertion into the tissue of the individual in need thereof; inserting the cannula into the individual; and delivering insulin via the cannula into the individual in need thereof; wherein the cannula is implanted in the individual in need thereof for at least 3 continuous days before the cannula is replaced or removed.

In some embodiments, the disclosure provides a method of administering insulin to an individual in need thereof, comprising: providing a cannula, wherein the cannula comprises a copolymer, and the copolymer is polycarbonate polyurethane polysiloxane (PC-PU-PS), wherein the cannula is configured for subcutaneous insertion into the tissue of the individual in need thereof; inserting the cannula into the individual; and delivering insulin via the cannula into the individual in need thereof; wherein the cannula is implanted in the individual in need thereof for at least 3 continuous days before the cannula is replaced or removed, and the average total daily dose (TDD) of insulin delivered to the individual in need thereof is about or less than the average TDD of insulin delivered via an analogous cannula comprising PTFE or FEP, over a period of at least 3 continuous days.

The term "about" with regard to the dimensional values indicates values slightly outside the cited values, e.g., plus or minus 0.1% to 10%, plus or minus 0.1% to 5%, or plus or minus 0.1% to 2%. In some embodiments, the term "about" indicates values plus or minus 10% of the cited values. In some embodiments, the term "about" indicates values plus or minus 5% of the cited values. In some embodiments, the term "about" indicates values plus or minus 2% of the cited values. In some embodiments, the term "about" indicates values plus or minus 1% of the cited values.


III. Evaluating Cannula Performance


Examples of evaluating cannula performance include monitoring the total daily dose (TDD) of insulin and determining a patient's comfort level during cannula insertion or during insulin infusion.

The TDD is the amount of insulin needed to maintain an individual's blood glucose at a desirable level. Foreign body response, tissue injury, and infusion site-loss as a result of infusion device use (*i.e.* cannula implantation and insulin infusion) can diminish the effective delivery of insulin. When the body begins to react at the insertion site, the amount of insulin needed to maintain normal blood glucose levels of the patient will begin to increase. Eventually, no amount of insulin will reduce glucose levels, at which point the infusion set must be removed and a new site for infusion is needed. Hence, monitoring the TDD of insulin is useful in assessing the implanted cannula performance.

The subjective level of comfort and discomfort of cannula insertion can be assessed by providing test subjects with a questionnaire. Immediately following each cannula insertion, a comfort/discomfort Likert scale (very uncomfortable, uncomfortable, neutral, comfortable, and very comfortable) may be immediately presented to each study participant. The patient indicates which level is closest to that perceived during cannula penetration and the response is recorded. The word "pain" is not used in order to avoid influencing the perception of study participants. At the end of the test, the data is tabulated and analyzed in pairs ("conventional technique" x "emergent technique") with the aid of SPSS® software.

Where the context permits, singular or plural terms may also include the plural or singular term, respectively. Although specific embodiments of, and examples for, the technology are described above for illustrative purposes, various equivalent modifications are possible within the scope of the technology, as those skilled in the relevant art will recognize.

As used herein, the terms "generally," "substantially," "about," and similar terms are used as terms of approximation and not as terms of degree, and are intended to account for the inherent variations in measured or calculated values that would be recognized by those of ordinary skill in the art. As used herein, the term "about" is used synonymously with the term "approximately". Illustratively, the use of the term "about" with regard to an amount indicates values slightly outside the cited values, e.g., plus or minus 0.1% to 10%, plus or minus 0.1% to 5%, or plus or minus 0.1% to 2%.

Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the term "comprising" is used throughout to mean including at least the recited feature(s) such that any greater number of the same feature and/or additional types of other features are not precluded.

IV. UNDERLINE: EXAMPLE EMBODIMENTS

Some of the embodiments of this disclosure relate to Embodiment I, as follows:

Embodiment I-1. A cannula for use in delivering insulin to an individual in need thereof, wherein: the cannula comprises a copolymer, and the copolymer is polycarbonate polyurethane polysiloxane (PC-PU-PS); and the cannula is configured for subcutaneous insertion into the tissue of the individual in need thereof.

Embodiment I-2. The cannula according to Embodiment I-1, wherein the elastic stiffness of the cannula at 25 °C is higher than at 37 °C.

Embodiment I-3. The cannula according to Embodiment I-1, wherein the difference in elastic stiffness of the cannula at 25 °C and 37 °C is at least 25 MPa, at least 50 MPa, at least 75 MPa, at least 100 MPa, at least 150 MPa, at least 200 MPa, at least 250 MPa, at least 300 MPa, or at least 400 MPa, as measured by dynamic mechanical analysis (DMA) at a cycle rate of 0.8 Hz.

Embodiment I-4. The cannula according to any one of Embodiments I-1 to I-3, wherein the difference in elastic stiffness of the cannula at 25 °C and 37 °C is 300 MPa to 400 MPa, 250 MPa to 350 MPa, 200 to 300 MPa, 100-200 MPa, 50 to 100 MPa, or 25 to 75 MPa, as measured by DMA at a cycle rate of 0.8 Hz.

Embodiment I-5. The cannula according to any one of Embodiments I-1 to I-4, wherein the difference in elastic stiffness of the cannula at 25 °C and 37 °C is greater than the difference in elastic stiffness of PTFE at 25 °C and 37 °C, as measured by DMA at a cycle rate of 0.8 Hz.

Embodiment I-6. The cannula according to any one of Embodiments I-1 to I-5, wherein the elastic stiffness of the cannula at 25 °C is at least 20%, at least 50%, at least 75%, at least 100%, at least 125%, or at least 150% greater than the elastic stiffness of the copolymer at 37 °C, as measured by DMA at a cycle rate of 0.8 Hz.

Embodiment I-7. The cannula according to any one of Embodiments I-1 to I-6, wherein the cannula exhibits a tan delta peak between 30 °C and 50 °C, as measured by DMA at a cycle rate of 0.8 Hz.

Embodiment I-8. The cannula according to any one of Embodiments I-1 to I-7, wherein the tan delta peak of the cannula has an inflection point between 30 °C and 50 °C, as measured by DMA at a cycle rate of 0.8 Hz.

Embodiment I-9. The cannula according to any one of Embodiments I-1 to I-8, wherein the cannula further comprises a needle located within the cannula.

Embodiment I-10. The cannula according to any one of Embodiments I-1 to I-8, wherein the cannula further comprises a needle located on the exterior of the cannula.

Embodiment I-11. The cannula according to any one of Embodiments I-1 to I-10, wherein the cannula does not contain polyfluoroalkyl substances (PFAS).

Embodiment I-12. The cannula according to any one of Embodiments I-1 to I-11, wherein the cannula does not contain a plasticizer additive.

Embodiment I-13. The cannula according to any one of Embodiments I-1 to I-12, wherein the individual in need thereof is a diabetic patient.

Embodiment I-14. The cannula according to any one of Embodiments I-1 to I-14, wherein the copolymer is selected from the group consisting of ARCS-70A, ARCS-75A, ARCS-80A, ARCS-85A, ARCS-90A, ARCS-95A, ARCS-55D, ARCS-60D, and ARCS-70D, or a combination thereof.

Embodiment I-15. The cannula according to any one of Embodiments I-1 to I-14, wherein the copolymer is selected from the group consisting of ARCS-90A, ARCS-95A, ARCS-55D, ARCS-60D, and ARCS-70D, or a combination thereof.

Embodiment I-16. The cannula according to any one of Embodiments I-1 to I-15, wherein the copolymer has a physical property profile equivalent to the physical property profile of an ARCS copolymer selected from the group consisting of ARCS-70A, ARCS-75A, ARCS-80A, ARCS-85A, ARCS-90A, ARCS-95A, ARCS-55D, ARCS-60D, and ARCS-70D, wherein the ARCS copolymer physical property profile is listed in Table 1.

Embodiment I-17. The cannula according to any one of Embodiments I-1 to I-15, wherein: the copolymer has a physical property profile equivalent to the physical property profile of an ARCS copolymer selected from the group consisting of ARCS-90A, ARCS-95A, ARCS-55D, ARCS-60D, and ARCS-70D; and the ARCS copolymer physical property profile is listed in Table 1.

Embodiment I-18. The cannula according to any one of Embodiments I-1 to I-15, wherein: the copolymer has a physical property profile comprising two or more physical properties selected from the group consisting of durometer hardness, specific gravity, flex modulus, ultimate tensile, ultimate elongation tensile at 100%, tensile at 300%, and mold shrinkage; and the physical property profile is equivalent to the physical property profile of an ARCS copolymer described in Table 1.

Embodiment I-19. The cannula according to any one of Embodiments I-1 to I-15, wherein: the copolymer has a physical property profile comprising two or more physical properties selected from the group consisting of durometer hardness, specific gravity, flex modulus, ultimate tensile, ultimate elongation tensile at 100%, tensile at 300%, and mold shrinkage; and the physical property profile is equivalent to the physical property profile of a copolymer selected from the group consisting of ARCS-90A, ARCS-95A, ARCS-55D, ARCS-60D, and ARCS-70D, as described in Table 1.

Embodiment I-20. The cannula according to any one of Embodiments I-1 to I-19, wherein the distal end of the cannula comprises a tip capable of penetrating the skin of the individual in need thereof.

Embodiment I-21. The cannula according to any one of Embodiments I-1 to I-20, wherein two or more, three or more, or four or more of the dimensions of the cannula are proportional to the dimensions of the cannula described in Table 4.

Embodiment I-22. The cannula according to any one of Embodiments I-1 to I-21, wherein two or more, three or more, or four or more of the dimensions of the cannula are about the dimensions of the cannula described in Table 4.

Embodiment I-23. A method of administering insulin to an individual in need thereof, comprising: providing a cannula, wherein the cannula comprises a copolymer, and the copolymer is polycarbonate polyurethane polysiloxane (PC-PU-PS), wherein the cannula is configured for subcutaneous insertion into the tissue of the individual in need thereof; inserting the cannula into the individual; and delivering insulin via the cannula into the individual in need thereof.

Embodiment I-24. The method according to Embodiment I-23, wherein the cannula is inserted in subcutaneous tissue on the abdomen, thighs, hips, upper arms, lower back, or buttocks of the individual in need thereof.

Embodiment I-25. The method according to Embodiment I-23 or I-24, wherein the cannula resists bunching or kinking during insertion into the individual in need thereof.

Embodiment I-26. The method according to any one of Embodiments I-23 to I-25, wherein the cannula is implanted in the individual in need thereof for at least 3, at least 4, at least 5, at least 6, at least 7, at least 8, at least 9, or at least 10 continuous days before the cannula is replaced or removed.

Embodiment I-27. The method according to any one of Embodiments I-23 to I-26, wherein: the individual in need thereof experiences greater comfort, reduced inflammation, or reduced foreign body response compared to an analogous cannula comprising PTFE or FEP.

Embodiment I-28. The method according to Embodiment I-27, wherein the greater comfort, reduced inflammation, or reduced foreign body response compared to an analogous cannula comprising PTFE or FEP is associated with the decreased stiffness of the cannula at body temperature.

Embodiment I-29. The method according to any one of Embodiments I-23 to I-28, wherein the average total daily dose (TDD) of insulin delivered to the individual in need thereof is about or less than the average TDD of insulin delivered via an analogous cannula comprising PTFE or FEP, over a period of at least 3, at least 4, at least 5, at least 6, at least 7, at least 8, at least 9, or at least 10 continuous days.

Embodiment I-30. The method according to any one of Embodiments I-23 to I-29, wherein the average TDD of insulin delivered to an individual in need thereof is about or less than the average TDD of insulin delivered to an individual in need thereof via the analogous cannula comprising PTFE or FEP, over a period of up to 10 continuous days.

Embodiment I-31. The method according to any one of Embodiments I-23 to I-30, wherein the TDD of insulin delivered to an individual in need thereof does not increase by more than at least 25% than the average TDD over a period of up to 10 continuous days.

Embodiment I-32. The method according to any one of Embodiments I-23 to I-31, wherein the cannula is in fluid connection with an insulin infusion device.

Embodiment I-33. The method according to any one of Embodiments I-23 to I-32, wherein the elastic stiffness of the cannula at 25 °C is higher than at 37 °C.

Embodiment I-34. The method according to any one of Embodiments I-23 to I-33, wherein the difference in elastic stiffness of the cannula at 25 °C and 37 °C is at least 25 MPa, at least 50 MPa, at least 75 MPa, at least 100 MPa, at least 150 MPa, at least 200 MPa, at least 250 MPa, at least 300 MPa, or at least 400 MPa, as measured by DMA at a cycle rate of 0.8 Hz.

Embodiment I-35. The method according to any one of Embodiments I-23 to I-34, wherein the difference in elastic stiffness of the cannula at 25 °C and 37 °C is 300 MPa to 400 MPa, 250 MPa to 350 MPa, 200 to 300 MPa, 100-200 MPa, 50 to 100 MPa, or 25 to 75 MPa, as measured by DMA at a cycle rate of 0.8 Hz.

Embodiment I-36. The method according to any one of Embodiments I-23 to I-35, wherein the difference in elastic stiffness of the cannula at 25 °C and 37 °C is greater than the difference in elastic stiffness of PTFE at 25 °C and 37 °C.

Embodiment I-37. The method according to any one of Embodiments I-23 to I-36, wherein the elastic stiffness of the cannula at 25 °C is at least 20%, at least 50%, at least 75%, at least 100%, at least 125%, or at least 150% greater than the elastic stiffness of the copolymer at 37 °C.

Embodiment I-38. The method according to any one of Embodiments I-23 to I-37, wherein the cannula exhibits a tan delta peak between 30 °C and 50 °C, as measured by DMA at a cycle rate of 0.8 Hz.

Embodiment I-39. The method according to any one of Embodiments I-23 to I-38, wherein the tan delta peak of the cannula has an inflection point between 30 °C and 50 °C, as measured by DMA at a cycle rate of 0.8 Hz.

Embodiment I-40. The method according to any one of Embodiments I-23 to I-39, wherein the cannula further comprises a needle located within the cannula.

Embodiment I-41. The method according to any one of Embodiments I-23 to I-39, wherein the cannula further comprises a needle located on the exterior of the cannula.

Embodiment I-42. The method according to any one of Embodiments I-23 to I-41, wherein the cannula does not contain polyfluoroalkyl substances (PFAS).

Embodiment I-43. The method according to any one of Embodiments I-23 to I-42, wherein the cannula does not contain a plasticizer additive.

Embodiment I-44. The method according to any one of Embodiments I-23 to I-43, wherein the individual in need thereof is a diabetic patient.

Embodiment I-45. The method according to any one of Embodiments I-23 to I-44, wherein the copolymer is selected from the group consisting of ARCS-70A, ARCS-75A, ARCS-80A, ARCS-85A, ARCS-90A, ARCS-95A, ARCS-55D, ARCS-60D, and ARCS-70D, or a combination thereof.

Embodiment I-46. The method according to any one of Embodiments I-23 to I-44, wherein the copolymer is selected from the group consisting of ARCS-90A, ARCS-95A, ARCS-55D, ARCS-60D, and ARCS-70D, or a combination thereof.

Embodiment I-47. The method according to any one of Embodiments I-23 to I-46, wherein the copolymer has a physical property profile equivalent to the physical property profile of an ARCS copolymer selected from the group consisting of ARCS-70A, ARCS-75A, ARCS-80A, ARCS-85A, ARCS-90A, ARCS-95A, ARCS-55D, ARCS-60D, and ARCS-70D, wherein the ARCS copolymer physical property profile is listed in Table 1.

Embodiment I-48. The method according to any one of Embodiments I-23 to I-46, wherein: the copolymer has a physical property profile equivalent to the physical property profile of an ARCS copolymer selected from the group consisting of ARCS-90A, ARCS-95A, ARCS-55D, ARCS-60D, and ARCS-70D; and the ARCS copolymer physical property profile is listed in Table 1.

Embodiment I-49. The method according to any one of Embodiments I-23 to I-46, wherein: the copolymer has a physical property profile comprising two or more physical properties selected from the group consisting of durometer hardness, specific gravity, flex modulus, ultimate tensile, ultimate elongation tensile at 100%, tensile at 300%, and mold shrinkage; and the physical property profile is equivalent to the physical property profile of an ARCS copolymer described in Table 1.

Embodiment I-50. The method according to any one of Embodiments I-23 to I-46, wherein: the copolymer has a physical property profile comprising two or more physical properties selected from the group consisting of durometer hardness, specific gravity, flex modulus, ultimate tensile, ultimate elongation tensile at 100%, tensile at 300%, and mold shrinkage; and the physical property profile is equivalent to the physical property profile of a copolymer selected from the group consisting of ARCS-90A, ARCS-95A, ARCS-55D, ARCS-60D, and ARCS-70D, as described in Table 1.

Embodiment I-51. The method according to any one of Embodiments I-23 to I-50, wherein the distal end of the cannula comprises a tip capable of penetrating the skin of the individual in need thereof.

Embodiment I-52. The method according to any one of Embodiments I-23 to I-51, wherein two or more, three or more, or four or more of the dimensions of the cannula are proportional to the dimensions of the cannula described in Table 4.

Embodiment I-53. The method according to any one of Embodiments I-23 to I-52, wherein two or more, three or more, or four or more of the dimensions of the cannula are about the dimensions of the cannula described in Table 4.

Embodiment I-54. An infusion device comprising: a housing configured to be positioned at the skin of a patient at an infusion site; a reservoir configured to store a fluid medication, the reservoir configured to be received by the housing; and a cannula configured for subcutaneous insertion into a tissue of the patient at the infusion site, wherein the cannula is a cannula according to any one of Embodiments I-1 to I-22.

V. <u>EXAMPLES</u>

The following specific examples are to be construed as merely illustrative, and not limitative of the remainder of the disclosure in any way whatsoever.

Example 1: General Process for Preparing Cannulas

This example describes a general process for preparing cannulas described herein.

Quadrasil™ ARCS resin (PC-PU-PS) was obtained from Biomerics and added to a feed hopper of a screw extruder machine. The catheter was extruded on conventional equipment and specific processing parameters related to the particular resin was obtained from the supplier. The extruded catheter was cut to length and flared onto a mandrel mounted bushing by interference fitting. The distal end of the catheter was then tip-formed using a heated and lubricated mold. The process required pre-heating a tip-forming mold to a predetermined temperature in order to reflow the resin and force the tip of the catheter to conform to the shape of the mold. A silicone/siloxane lubricant was applied to the heated mold surfaces just prior to tip-forming. The distal end of the catheter is forced and pressed into the heated mold. A mandrel was used to hold, guide, and center the cannula with the heated mold during tip-forming. The distal end was held in the mold for a predetermined dwell time to allow the distal end of the cannula in contact with the mold to reflow and conform to the dimensions of the mold. After the predetermined dwell time was completed, the cannula was retracted from the mold.

Example 2: Comparison of Elastic Stiffness of Various Cannula Materials

This example compares the elastic stiffness of three different materials at temperatures from 25 °C to 60 °C.

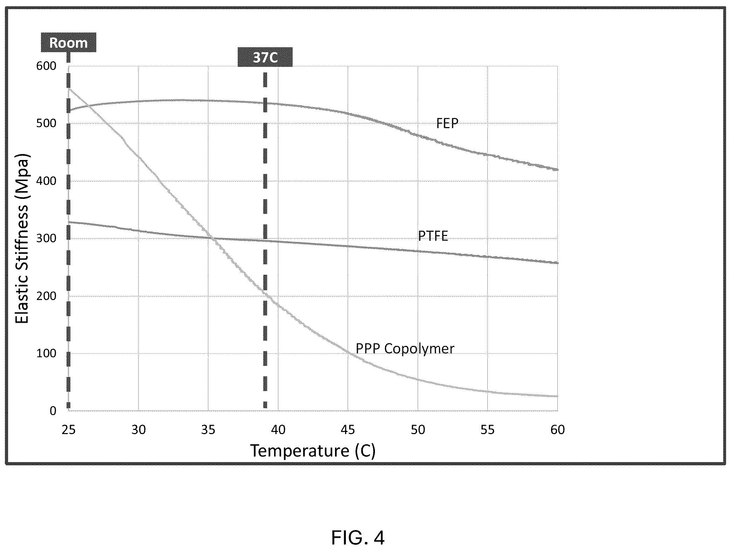

Samples of PTFE, FEP, and Quadrasil™ ARCS resin (PC-PU-PS) was subjected to DMA at a cycle rate 0.8 Hz while the temperature of the sample was increased from 25 °C to 60 °C. The elastic stiffness was calculated in MPa for each sample and plotted as a function of temperature, as shown in FIG. 4.

FEP has an elastic stiffness of approximately 525 MPa at 25 °C, which represents room temperature, and does not fluctuate appreciably at 37 °C, which represents human body temperature. PTFE has an elastic stiffness of about 325 MPa at 25 °C and decreases to just under 300 MPa at 37 °C. The higher elastic stiffness values for FEP suggests that it is stiffer than PTFE. It is noted that FEP does not perform as well as PTFE *in vivo,* which might be due to FEP's stiffness at body temperature.

The elastic stiffness curve for the PC-PU-PS material is significantly different from the two PFAS polymers. At room temperature, PC-PU-PS has an elastic stiffness of about 550 MPa, which is higher than both FEP and PTFE. The stiffness value decreases to 200 MPa at body temperature, which is about 100 MPa lower than PTFE. This suggests that PC-PU-PS is stiffer than PTFE and FEP at room temperature but softer than PTFE and FEP at body temperature. The high mechanical stiffness of PC-PU-PS at room temperature can help minimize failures due to cannula bunching or kinking during the insertion process. The softer and more flexible character of the implanted cannula may increase comfort for the patient, reduce inflammation, and foreign body response to the cannula. As a result of a reduction in inflammation and foreign body response, the implanted cannula may have a longer duration of viability, which means the patient can wear the cannula for a longer period of time before replacing it with a fresh cannula.

Based on the results discussed above, the mechanical behavior of the PC-PU-PS copolymer is superior to the PTFE and FEP polymers that are currently used as infusion cannulas.

Example 3: Comparison of PTFE and PC-PU-PS

This example compares the mechanical behavior of PC-PU-PS to PTFE cannula material.

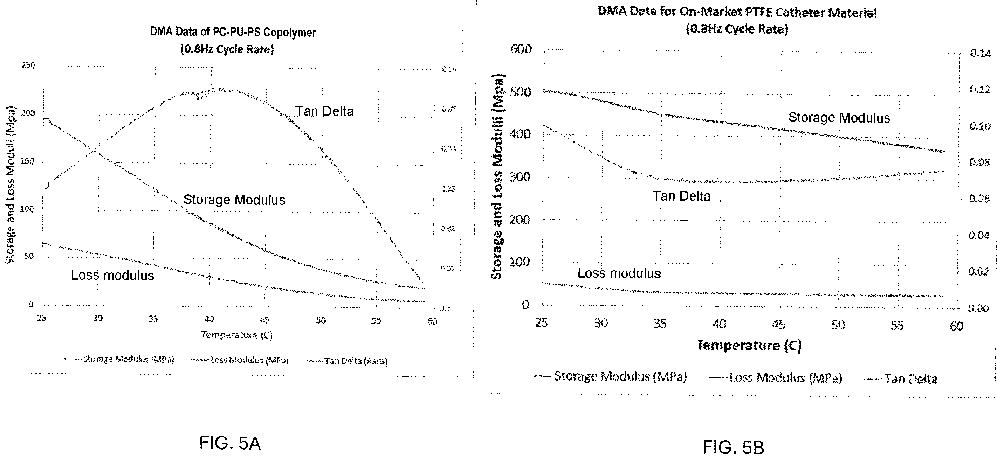

Samples of PC-PU-PS and PTFE cannula material were obtained and subjected to DMA at 0.8 Hz cycle rate. The storage modulus, loss modulus, and tan delta were calculated based on the DMA measurements and plotted as a function of temperature. FIG. 5A shows the data plot for PC-PU-PS. The tan delta peaks around 40 °C, which is very close to body temperature of 37 °C.

An increasing tan delta indicates greater dissipation of elastic energy and lower elasticity, which is ideal for an implanted cannula. The greater the tan delta means that the material is less likely to "spring back" when deformed and flexed in the body. This flexibility and lowered elasticity is likely to improve comfort and reduce an inflammatory or foreign body response, which in turn leads to longer wear times for the patient and fewer times the cannula needs to be replaced.

FIG. 5B shows the data plot for PTFE, which does not exhibit a tan delta peak or inflection point near body temperature. This means that the PTFE material will be less likely to deform and flex with the motion of the body. A rigid implanted cannula potentially increases the risk of tissue trauma, inflammation, and foreign body response; events that decrease the longevity of the cannula and infusion site.

The tan delta profile of the PC-PU-PS is surprising especially since the copolymer contains a polycarbonate component, which is known to be a rigid, stiff material. For the purpose of a subcutaneous infusion cannula that is rigid at insertion and then softens at body temperature, the tan delta peak of the PC-PU-PS suggests superior mechanical behavior compared to PTFE.

Example 3: Preclinical Testing of Cannulas in a Diabetic Porcine Model

The performance of three types of PTFE-free cannulas were tested in a diabetic porcine model and compared to a conventional PTFE cannula over a period of 10 days. Commercially available infusion devices were modified to accommodate the test cannulas. The PFAS-free test cannulas were prepared from ARCS-95A (n=4), ARCS-70D (n=4), or ARCS-55 (n=4), where n indicates the number of pig subjects tested. The cannula made from PTFE (n=4) was part of the commercially available Medtronic Extended™ Infusion Set (EIS).

The animal subjects were fasted for over 12 hours prior to testing. On Day 0, the pigs were fed a standardized diet at prescribed times. A cannula was placed subcutaneously into abdominal tissue while the subject was under general anesthesia. A glucose sensor was also implanted at least 5 cm from the cannula. In addition, blood glucose levels were measured 5 times daily at prescribed times (*i.e.* 07:30, 09:30, 11:30, 14:30, and 16:30). The infusion device was used to deliver Novolog insulin into the subjects.

The basal rate of delivery was established for Day 0, which represented the basal rate of delivery needed to maintain blood glucose concentrations in the range of 100 - 400 mg/dL. The infusion device delivered bolus amounts of insulin as needed, for instance after meals when the blood glucose concentration increases beyond the desired range. The infusion set was considered failed when the blood glucose concentration does not respond to an insulin dose change, for instance when administering increasing amounts of insulin fail to reduce or control blood glucose concentrations.

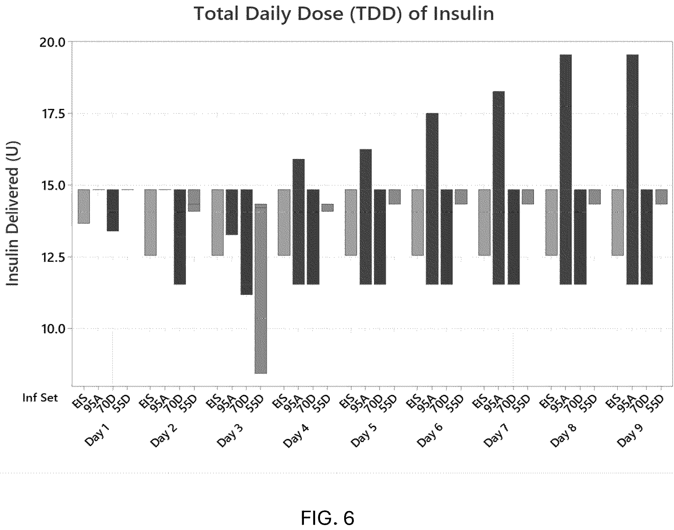

The amount of insulin administered over a 24-hour period, Total Daily Dose (TDD), was calculated for a nine-day period or until the infusion set failed. Table 2 summarizes the TDD for each test group and the p-value of the test group compared to the PTFE group from Day 1 through Day 9. FIG. 6 is a graphical summary of the TDD for each test group over nine days. The experiment was concluded on Day 10.

Table 2.

| Group | Parameter | Day 1 | Day 2 | Day 3 | Day 4 | Day 5 | Day 6 | Day 7 | Day 8 | Day 9 |
|---|---|---|---|---|---|---|---|---|---|---|
| EIS (n=4) | TDD, U/day | 14.55 ± 0.59 | 14.27 ± 1.15 | 14.27 ± 1.15 | 14.27 ± 1.15 | 14.27 ± 1.15 | 14.27 ± 1.15 | 14.27 ± 1.15 | 14.27 ± 1.15 | 14.27 1.15 |
| ARCS-95A (n=4) | TDD, U/day | 14.85 ± 0.00 | 14.85 ± 0.00 | 14.46 ± 0.79 | 14.29 ± 1.90 | 14.38 ± 2.00 | 14.69 ± 2.44 | 14.88 ± 2.74 | 15.20 ± 3.29 | 15.20 ± 3.29 |
| | P Value vs. EIS | 0.356 | 0.356 | 0.804 | 0.989 | 0.934 | 0.770 | 0.699 | 0.615 | 0.615 |
| ARCS-70D (n=3) | TDD, U/day | 13.88 ± 0.84 | 12.65 ± 1.91 | 12.65 ± 1.91 | 12.65 ± 1.91 | 12.65 ± 1.91 | 12.65 ± 1.91 | 12.65 ± 1.91 | 12.65 ± 1.91 | 12.65 1.91 |
| | P Value vs. EIS | 0.266 | 0.215 | 0.215 | 0.215 | 0.215 | 0.215 | 0.215 | 0.215 | 0.215 |
| ARCS-55D (n=3) | TDD, U/day | 14.85 ± 0.00 | 14.41 ± 0.31 | 12.81 ± 2.91 | 14.27 ± 0.14 | 14.52 ± 0.29 | 14.52 ± 0.29 | 14.52 ± 0.29 | 14.52 ± 0.29 | 14.52 0.29 |
| | P Value vs. EIS | 0.356 | 0.825 | 0.386 | 0.991 | 0.742 | 0.742 | 0.742 | 0.742 | 0.742 |

All the subjects in each test group survived the 10-day study. For each test group, the average TDD did not significantly vary over the duration of the experiment, which suggests that there was no appreciable deterioration of the infusion site. Cannulas made from PTFE, ARCS-95A, ARCS-55D, and ARCS-70D were successful in delivering insulin at a rate that maintained the desired blood glucose concentrations over the entire test period. This suggests that there was very little inflammation, no occlusions, or infusion site-loss as a result of the insulin infusion treatment. In addition, there was no statistically significant difference of TDD between the three test cannulas and PTFE. This data demonstrates that cannulas prepared from PC-PU-PS performed as well as the PTFE cannula *in vivo* over a period of nine days.

As previously discussed, polycarbonate is known to be a hard, stiff, and rigid material. Surprisingly, its incorporation in the PC-PU-PS copolymer did not hinder the performance of the test cannulas.

The average TDD change from Day 0 to Day 9 for PTFE, ARCS-95A, ARCS-55D, and ARCS-70D cannulas are summarized in Table 3.

Table 3.

| Cannula Material | Average TDD Change From Day 0 to Day 9 |
|---|---|
| PTFE | -4% |
| ACRS 95A Cannula | +2% |
| ACRS 55D Cannula | -2% |
| ACRS 70D Cannula | -15% |

Example 4: Example Cannula Specification

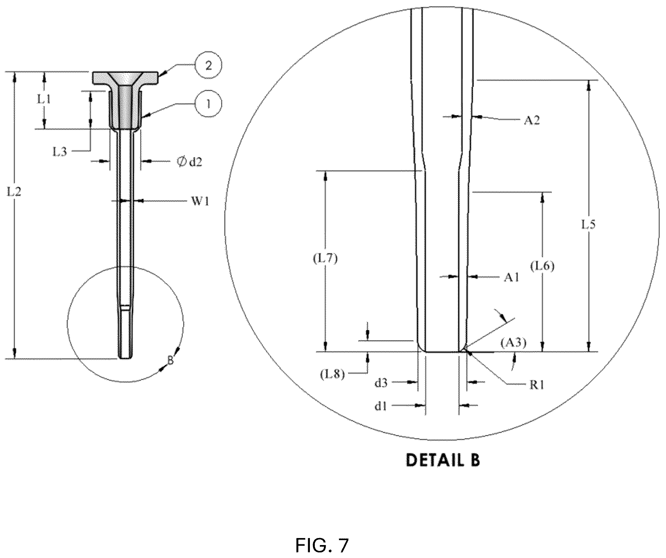

This example provides a dimensional description of an example cannula disclosed herein. Table 4 provides the dimensions of various parameters of the cannula shown in FIG. 7. Feature 1 shows the proximal end of the cannula flared onto a needle guide/bushing labeled as Feature 2, which provide a fluid inlet for the cannula.

Table 4.

| Dimension | Description | Dimensional Ranges |
|---|---|---|
| L1 | Overall Bushing Length On Bushing | 1.0 to 6.0mm |
| L2 | Overall Subassembly Length | 7.0 to 20.0mm |
| L3 | Flare Length | 0.5 to 5.0 mm |
| W1 | Catheter Tubing Wall Thickness | 0.05 to 0.5mm |
| d1 | Mandrel Diameter at Tip / Catheter Tip Inner Diameter | 0.1 to 1.0 mm |
| d3 | Catheter Tip Outer Diameter | 0.1 to 2.0 mm |
| R1 | Tip Forming Die Dimension: Catheter Tip Radius | 0.05 to 0.5 mm |
| L5 | Tip Forming Die Dimension: Overall Lead-In Taper Length | 0.5 to 5.0 mm |
| A1 | Primary Tip Angle | 1.0 to 5.0 deg |

In some embodiments, the disclosure provides a cannula having a dimensional profile comprising two or more, three or more, four or more, five or more, six or more or seven or more dimensions within the dimensional ranges in Table 4. In some embodiments, the cannula has dimensions that fall within all the dimensional ranges provided in Table 4.

Conclusion

While embodiments of the present invention have been shown and described herein, those skilled in the art will understand that such embodiments are provided by way of example only. Numerous variations, changes, and substitutions will now occur to those skilled in the art without departing from the invention. It should be understood that various alternatives to the embodiments of the invention described herein may be employed in practicing the invention. For example, while steps are presented in a given order, alternative embodiments may perform steps in a different order. The various embodiments described herein may also be combined to provide further embodiments. It is intended that the following claims define the scope of the invention and that methods and structures within the scope of these claims and their equivalents be covered thereby.

It will also be appreciated that specific embodiments have been described herein for purposes of illustration, but that various modifications may be made without deviating from the technology. Further, while advantages associated with certain embodiments of the technology have been described in the context of those embodiments, other embodiments may also exhibit such advantages, and not all embodiments need necessarily exhibit such advantages to fall within the scope of the technology. Accordingly, the present disclosure and associated technology can encompass other embodiments not expressly shown or described herein.

The following examples are illustrative of the techniques described herein.

Example 1: A cannula for use in delivering insulin to an individual in need thereof, wherein:

the cannula comprises a copolymer, and the copolymer is polycarbonate polyurethane polysiloxane (PC-PU-PS); and the cannula is configured for subcutaneous insertion into the tissue of the individual in need thereof.

Example 2: The cannula according to example 1, wherein the elastic stiffness of the cannula at 25 °C is higher than at 37 °C.

Example 3: The cannula according to example 1, wherein the difference in elastic stiffness of the cannula at 25 °C and 37 °C is at least 25 MPa, at least 50 MPa, at least 75 MPa, at least 100 MPa, at least 150 MPa, at least 200 MPa, at least 250 MPa, at least 300 MPa, or at least 400 MPa, as measured by dynamic mechanical analysis (DMA) at a cycle rate of 0.8 Hz.

Example 4: The cannula according to example 1, wherein the elastic stiffness of the cannula at 25 °C is at least 20%, at least 50%, at least 75%, at least 100%, at least 125%, or at least 150% greater than the elastic stiffness of the copolymer at 37 °C, as measured by DMA at a cycle rate of 0.8 Hz.

Example 5: The cannula according to example 1, wherein the cannula does not contain polyfluoroalkyl substances (PFAS).

Example 6: The cannula according to example 1, wherein the copolymer is selected from the group consisting of ARCS-70A, ARCS-75A, ARCS-80A, ARCS-85A, ARCS-90A, ARCS-95A, ARCS-55D, ARCS-60D, and ARCS-70D, or a combination thereof.

Example 7: The cannula according to example 1, wherein the copolymer has a physical property profile equivalent to the physical property profile of an ARCS copolymer selected from the group consisting of ARCS-70A, ARCS-75A, ARCS-80A, ARCS-85A, ARCS-90A, ARCS-95A, ARCS-55D, ARCS-60D, and ARCS-70D, wherein the ARCS copolymer physical property profile is listed in Table 1.

Example 8: The cannula according to example 1, wherein: the copolymer has a physical property profile comprising two or more physical properties selected from the group consisting of durometer hardness, specific gravity, flex modulus, ultimate tensile, ultimate elongation tensile at 100%, tensile at 300%, and mold shrinkage; and the physical property profile is equivalent to the physical property profile of an ARCS copolymer described in Table 1.

Example 9: The cannula according to example 1, wherein two or more, three or more, or four or more of the dimensions of the cannula are about the dimensions of the cannula described in Table 4.

Example 10: A method of administering insulin to an individual in need thereof, comprising

providing a cannula, wherein the cannula comprises a copolymer, and the copolymer is polycarbonate polyurethane polysiloxane (PC-PU-PS), wherein the cannula is configured for subcutaneous insertion into the tissue of the individual in need thereof;
inserting the cannula into the individual; and
delivering insulin via the cannula into the individual in need thereof.

Example 11: The method according to example 10, wherein the average total daily dose (TDD) of insulin delivered to the individual in need thereof is about or less than the average TDD of insulin delivered via an analogous cannula comprising PTFE or FEP, over a period of at least 3, at least 4, at least 5, at least 6, at least 7, at least 8, at least 9, or at least 10 continuous days.

Example 12: The method according to example 10, wherein the elastic stiffness of the cannula at 25 °C is higher than at 37 °C.

Example 13: The method according to example 10, wherein the difference in elastic stiffness of the cannula at 25 °C and 37 °C is at least 25 MPa, at least 50 MPa, at least 75 MPa, at least 100 MPa, at least 150 MPa, at least 200 MPa, at least 250 MPa, at least 300 MPa, or at least 400 MPa, as measured by DMA at a cycle rate of 0.8 Hz.

Example 14: The method according to example 10, wherein the elastic stiffness of the cannula at 25 °C is at least 20%, at least 50%, at least 75%, at least 100%, at least 125%, or at least 150% greater than the elastic stiffness of the copolymer at 37 °C.

Example 15: The method according to example 10, wherein the cannula exhibits a tan delta peak between 30 °C and 50 °C, as measured by DMA at a cycle rate of 0.8 Hz.

Example 16: The method according to example 10, wherein the cannula does not contain polyfluoroalkyl substances (PFAS).

Example 17: The method according to example 10, wherein the copolymer is selected from the group consisting of ARCS-70A, ARCS-75A, ARCS-80A, ARCS-85A, ARCS-90A, ARCS-95A, ARCS-55D, ARCS-60D, and ARCS-70D, or a combination thereof.

Example 18: The method according to example 10, wherein the copolymer has a physical property profile equivalent to the physical property profile of an ARCS copolymer selected from the group consisting of ARCS-70A, ARCS-75A, ARCS-80A, ARCS-85A, ARCS-90A, ARCS-95A, ARCS-55D, ARCS-60D, and ARCS-70D, wherein the ARCS copolymer physical property profile is listed in Table 1.

Example 19: The method according to example 10, wherein two or more, three or more, or four or more of the dimensions of the cannula are about the dimensions of the cannula described in Table 4.

Example 20: An infusion device comprising:

a housing configured to be positioned at the skin of a patient at an infusion site;
a reservoir configured to store a fluid medication, the reservoir configured to be received by the housing; and
a cannula configured for subcutaneous insertion into a tissue of the patient at the infusion site, wherein the cannula is a cannula according to claim 1.

**Claims**

1. A cannula for use in delivering insulin to an individual in need thereof, wherein:

   the cannula comprises a copolymer, and the copolymer is polycarbonate polyurethane polysiloxane (PC-PU-PS); and
   the cannula is configured for subcutaneous insertion into the tissue of the individual in need thereof.

2. The cannula according to claim 1, wherein the elastic stiffness of the cannula at 25 °C is higher than at 37 °C.

3. The cannula according to any one of claims 1-2, wherein the cannula does not contain polyfluoroalkyl substances (PFAS).

4. The cannula according to any one of claims 1-3, wherein the copolymer is selected from the group consisting of ARCS-70A, ARCS-75A, ARCS-80A, ARCS-85A, ARCS-90A, ARCS-95A, ARCS-55D, ARCS-60D, and ARCS-70D, or a combination thereof.

5. The cannula according to any one of claims 1-4, wherein the copolymer has a physical property profile equivalent to the physical property profile of an ARCS copolymer selected from the group consisting of ARCS-70A, ARCS-75A, ARCS-80A, ARCS-85A, ARCS-90A, ARCS-95A, ARCS-55D, ARCS-60D, and ARCS-70D, wherein the ARCS copolymer physical property profile is listed in Table 1.

6. The cannula according to any one of claims 1-5, wherein: the copolymer has a physical property profile comprising two or more physical properties selected from the group consisting of durometer hardness, specific gravity, flex modulus, ultimate tensile, ultimate elongation tensile at 100%, tensile at 300%, and mold shrinkage; and the physical property profile is equivalent to the physical property profile of an ARCS copolymer described in Table 1.

7. The cannula according to any one of claims 1-6, wherein two or more, three or more, or four or more of the dimensions of the cannula are about the dimensions of the cannula described in Table 4.

8. A method of administering insulin to an individual in need thereof, comprising:

   providing a cannula, wherein the cannula comprises a copolymer, and the copolymer is polycarbonate polyurethane polysiloxane (PC-PU-PS), wherein the cannula is configured for subcutaneous insertion into the tissue of the individual in need thereof;
   inserting the cannula into the individual; and
   delivering insulin via the cannula into the individual in need thereof.

9. The method according to claim 8, wherein the average total daily dose (TDD) of insulin delivered to the individual in need thereof is about or less than the average TDD of insulin delivered via an analogous cannula comprising PTFE or FEP, over a period of at least 3, at least 4, at least 5, at least 6, at least 7, at least 8, at least 9, or at least 10 continuous days.

10. The method according to any one of claims 8-9, wherein the elastic stiffness of the cannula at 25 °C is higher than at 37 °C.

11. The method according to any one of claims 8-10, wherein the cannula does not contain polyfluoroalkyl substances (PFAS).

12. The method according to any one of claims 8-11, wherein the copolymer is selected from the group consisting of ARCS-70A, ARCS-75A, ARCS-80A, ARCS-85A, ARCS-90A, ARCS-95A, ARCS-55D, ARCS-60D, and ARCS-70D, or a combination thereof.

13. The method according to any one of claims 8-12, wherein the copolymer has a physical property profile equivalent to the physical property profile of an ARCS copolymer selected from the group consisting of ARCS-70A, ARCS-75A, ARCS-80A, ARCS-85A, ARCS-90A, ARCS-95A, ARCS-55D, ARCS-60D, and ARCS-70D, wherein the ARCS copolymer physical property profile is listed in Table 1.

14. The method according to any one of claims 8-13, wherein two or more, three or more, or four or more of the dimensions of the cannula are about the dimensions of the cannula described in Table 4.

15. An infusion device comprising:

a housing configured to be positioned at the skin of a patient at an infusion site;
a reservoir configured to store a fluid medication, the reservoir configured to be received by the housing; and
a cannula configured for subcutaneous insertion into a tissue of the patient at the infusion site, wherein the cannula is a cannula according to any one of claims 1-7.

FIG. 1

**1. Interface :**
- Needle guide used to prevent inadvertent damage to the cannula from the needle during assembly

**2. Materials :**
- Stiff during insertion for reliable penetration
- Soft in the body after insertion to minimize inflammation and injury
- Low friction for insertion reliability and minimal tissue trauma
- Coating (silicone oil) reduces effects of micromotion and increases longevity

**3. Biological Behavior:**
- Material unreactive to tissue
- Surface microstructure minimizes inflammatory response

**4. Process (Flaring):**
- Material compliant enough to flare
- Material elastic enough to form a fluid-tight seal

**5. Geometry (Length) :**
- Sufficiently long to reliably penetrate Sub-Q.
- Sufficiently short to avoid reaching muscle tissue.

**Quality of the tip is important for penetration and longevity of the catheter**

**6. Process & Geometry (Tipping):**
- Tip geometry optimized for minimal penetration force and pain
- Material compliant enough to tip into optimal geometry
- Tip must not be too sharp or too blunt for in-vivo longevity

**7. Fluid Path:**
- Material unreactive to insulin
- Opening sufficiently large to deliver without occlusions
- Opening sufficiently small to minimize tissue trauma
- Fill volume minimized to avoid wasted insulin

FIG. 2

FIG. 3

FIG. 4

**DMA Data for On-Market PTFE Catheter Material (0.8Hz Cycle Rate)**

Storage Modulus

Tan Delta

Loss modulus

Temperature (C)

Storage and Loss Modulii (MPa)

— Storage Modulus (MPa)   — Loss Modulus (MPa)   — Tan Delta

FIG. 5B

**DMA Data of PC-PU-PS Copolymer (0.8Hz Cycle Rate)**

Tan Delta

Storage Modulus

Loss modulus

Temperature (C)

Storage and Loss Modulii (Mpa)

— Storage Modulus (MPa)   — Loss Modulus (MPa)   — Tan Delta (Rads)

FIG. 5A

Total Daily Dose (TDD) of Insulin

FIG. 6

EP 4 643 911 A1

DETAIL B

FIG. 7

EP 4 643 911 A1

Europäisches
Patentamt
European
Patent Office
Office européen
des brevets

# EUROPEAN SEARCH REPORT

**Application Number**

EP 25 17 2873

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/399454 A1 (MUSE JAY ALLEN [US] ET AL) 14 December 2023 (2023-12-14)<br>* figure 1 *<br>* paragraphs [0018] - [0022], [0043], [0051], [0053], [0148], [0149], [0170] * | 1-15 | INV.<br>A61M5/158<br>A61L29/06<br>C08L75/04 |
| | ----- | | |
| | | | **TECHNICAL FIELDS SEARCHED (IPC)**<br><br>A61M<br>A61L<br>C08L |

The present search report has been drawn up for all claims

| Place of search | Date of completion of the search | Examiner |
|---|---|---|
| Munich | 25 June 2025 | López García, Mónica |

EPO FORM 1503 03.82 (P04C01)

## ANNEX TO THE EUROPEAN SEARCH REPORT
## ON EUROPEAN PATENT APPLICATION NO.

EP 25 17 2873

This annex lists the patent family members relating to the patent documents cited in the above-mentioned European search report.
The members are as contained in the European Patent Office EDP file on
The European Patent Office is in no way liable for these particulars which are merely given for the purpose of information.

25-06-2025

| Patent document cited in search report | Publication date | Patent family member(s) | Publication date |
|---|---|---|---|
| US 2023399454 A1 | 14-12-2023 | CN 111655761 A | 11-09-2020 |
| | | EP 3710512 A1 | 23-09-2020 |
| | | JP 7345485 B2 | 15-09-2023 |
| | | JP 2021505747 A | 18-02-2021 |
| | | KR 20200087826 A | 21-07-2020 |
| | | US 2023399454 A1 | 14-12-2023 |
| | | WO 2019099964 A1 | 23-05-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63641349

- US 19092565 B